# FASCICULE DE BREVET EUROPEEN

(11) **EP 4 125 741 B1**
(45) Date de publication et mention de la délivrance du brevet: **10.04.2024**
(21) Numéro de dépôt: 21720823.0
(22) Date de dépôt: 31.03.2021
(51) Int. Cl.: A61F 5/00

(54) **DISPOSITIF D'IMPLANTATION D'UN BALLON INTRA-GASTRIQUE GONFLABLE ET SYSTÈME DE TRAITEMENT DE L'OBÉSITÉ D'UN INDIVIDU COMPRENANT UN TEL DISPOSITIF D'IMPLANTATION**
VORRICHTUNG ZUR IMPLANTATION EINES AUFBLASBAREN INTRAGASTRISCHEN BALLONS UND SYSTEM ZUR BEHANDLUNG VON ADIPOSITAS BEI EINER PERSON MIT SOLCH EINER IMPLANTATIONSVORRICHTUNG
DEVICE FOR IMPLANTING AN INFLATABLE INTRA-GASTRIC BALLOON AND SYSTEM FOR TREATING OBESITY IN AN INDIVIDUAL COMPRISING SUCH AN IMPLANTATION DEVICE

(30) Priorité: 02.04.2020 FR 2003327
(43) Date de publication de la demande: 08.02.2023
(73) Titulaire: Medical Innovation Developpement, 69570 Dardilly (FR)
(72) Inventeur: CAZENAVE, Ludovic, 69005 LYON (FR); CLAIR, Yannis, 38290 LA VERPILLIERE (FR)
(74) Mandataire: Plasseraud IP
(86) Numéro de dépôt international: PCT/FR2021/050569
(87) Numéro de publication internationale: WO 2021/198618

(56) Documents cités:
- US-A- 4 416 267
- US-A1- 2017 333 239
- US-B2- 8 430 895

## Description

### Domaine technique

La présente invention se rapporte à un dispositif d'implantation d'un ballon intra-gastrique gonflable destiné à être introduit dans l'estomac d'un individu et à un système de traitement de l'obésité comprenant un tel dispositif d'implantation.

### Technique antérieure

Depuis ces dernières années, on assiste à une augmentation de l'obésité dans la population mondiale. Un des traitements connus pour le traitement des individus atteints d'obésité est la pose d'un ballon intra-gastrique gonflable dans l'estomac.

Le ballon intra-gastrique peut notamment être mis en place par voie endoscopique. Le ballon intra-gastrique dégonflé est positionné au bout d'un canal, par exemple un cathéter, et le canal est ensuite inséré dans la bouche, puis l'œsophage de l'individu atteint d'obésité jusqu'à ce que le ballon intra-gastrique atteigne l'estomac de l'individu. Le ballon intra-gastrique est alors gonflé à l'aide de sérum physiologique, d'air ou d'un mélange des deux, envoyé par l'intermédiaire du canal.

Une fois gonflé, le ballon intra-gastrique est dissocié du canal et est implanté dans l'estomac, notamment au niveau du fundus.

US4416267A décrit un implant ou insert stomacal pour traiter l'obésité chez l'homme en réduisant le volume de l'estomac. Cet insert comprend un ballon gonflable flexible en forme de tore, traversé par une ouverture centrale. Le ballon comprend un site d'injection 18 qui sert de point de gonflage du ballon, et qui est dimensionné de manière à ce que son volume gonflé soit d'environ 200 à 800 cc. Un tube d'insufflation en polyéthylène de petit diamètre est fixé au ballon dégonflé. L'extrémité libre du tube d'insufflation porte une aiguille qui perfore le ballonnet au niveau du site d'injection.
Une sonde gastrique est utilisée pour positionner ce ballon à l'intérieur de l'estomac. Le ballon stomacal dégonflé et le tube d'insufflation qui lui est attaché sont stockés à l'intérieur de la sonde stomacale juste avant l'introduction de la sonde stomacale par la bouche et dans l'estomac de la personne traitée pour l'obésité. Cette sonde stomacale tubulaire ouverte à ces deux extrémités ne permet pas d'assurer un positionnement simple et précis du ballon intragastrique dans l'estomac.

Ce traitement est facile à mettre en place et peu invasif. Cependant, il convient de trouver un système permettant d'améliorer le suivi de la dissociation du ballon intra-gastrique du canal, et permettant de s'assurer du bon positionnement du ballon intra-gastrique dans l'estomac, par exemple dans le fundus.

### Résumé

La présente invention vise à répondre au besoin évoqué ci-dessus.

A cet effet, selon un premier aspect, l'invention prévoit un dispositif d'implantation d'un ballon intra-gastrique gonflable pour implanter le ballon intra-gastrique dans un estomac, selon la revendication 1.

Ainsi, la coopération entre l'embout du canal et le ballon intra-gastrique permet d'assurer un détachement simplifié du ballon intra-gastrique du canal.

De plus, l'ouverture latérale permet de garantir le bon positionnement du ballon intra-gastrique dans l'estomac lorsque le ballon intra-gastrique est détaché du canal. En effet, l'ouverture latérale permet de contrôler la direction de largage du ballon intra-gastrique dans l'estomac et permet ainsi de le diriger vers le fundus.

La mise en place du ballon intra-gastrique dans l'estomac est ainsi facilitée.

Selon une variante de l'invention, le canal comprend un fourreau attaché à l'extrémité de raccordement de la gaine et un cathéter s'étendant à l'intérieur du fourreau, le cathéter étant monté coulissant par rapport au fourreau et comportant une extrémité proximale configurée pour être alimentée en fluide et une extrémité distale, l'extrémité distale comportant l'embout et étant en saillie du fourreau dans la position de gonflage.

Le cathéter peut coulisser dans le fourreau pour faire sortir l'embout de la cavité permettant ainsi de positionner le ballon intra-gastrique sur l'embout lorsque celui-ci est hors de la cavité. La mise en place du ballon intra-gastrique sur l'embout est donc facilitée. De plus, le cathéter peut également coulisser dans le fourreau pour permettre au ballon intra-gastrique d'être détaché de l'embout.

Selon une variante de l'invention, la paroi latérale de la gaine présente une surface d'appui, opposée à l'ouverture latérale et configurée pour que le ballon intra-gastrique prenne appui sur ladite surface d'appui lorsque ledit ballon intra-gastrique passe de l'état dégonflé à l'état gonflé.

La surface d'appui guide ainsi le ballon intra-gastrique vers l'ouverture latérale.

Selon une variante, l'ouverture latérale de la gaine présente un bord d'appui configuré pour que le ballon intra-gastrique prenne appui sur ledit bord d'appui pour permettre au ballon intra-gastrique d'être détaché de l'embout.

Le ballon intra-gastrique peut ainsi être détaché de l'embout sans que le ballon intra-gastrique ne prenne appui sur la paroi de l'estomac.

Selon une variante, dans la position de gonflage, l'embout s'étend selon l'axe longitudinal et est positionné entre l'extrémité de raccordement de la gaine et l'ouverture latérale de la gaine selon l'axe longitudinal.

Cette position de l'embout permet d'optimiser le positionnement du ballon intra-gastrique dans la cavité lorsque celui-ci passe de l'état dégonflé à l'état gonflé, afin de permettre au ballon intra-gastrique de sortir automatiquement de la gaine par l'ouverture latérale.

Selon une variante, le dispositif d'implantation est spécialement adapté pour un ballon intra-gastrique comportant une valve configurée pour coopérer avec l'embout du canal et présentant un état passant permettant de faire passer le fluide et un état bloquant permettant de bloquer le fluide, et l'embout est configuré pour être emmanché de façon amovible dans la valve du ballon intra-gastrique.

L'emmanchement facilite le positionnement du ballon intra-gastrique sur l'embout à l'état dégonflé tout en garantissant le détachement du ballon intra-gastrique à l'état gonflé.

Le cas échéant, l'embout peut présenter un axe d'embout et comprendre au moins un épaulement s'étendant transversalement par rapport à l'axe d'embout.

L'épaulement permet de participer au maintien du ballon intra-gastrique sur l'embout à l'état dégonflé et lorsque le ballon intra-gastrique passe de l'état dégonflé à l'état gonflé, tout en permettant le détachement du ballon intra-gastrique à l'état gonflé.

Selon une variante, l'ouverture latérale a une forme oblongue selon l'axe longitudinal.

Selon une variante, le dispositif d'implantation est spécialement adapté pour un ballon intra-gastrique sphérique présentant un diamètre à l'état gonflé, et la cavité de la gaine et l'ouverture latérale présentent chacune une longueur prise selon l'axe longitudinal et une largeur prise perpendiculairement à l'axe longitudinal telles que :
- un rapport du diamètre du ballon intra-gastrique sur la longueur de la cavité de la gaine est compris entre 0,44 et 0,83,
- un rapport du diamètre du ballon intra-gastrique sur la largeur de la cavité de la gaine est compris entre 3,2 et 6,7,
- un rapport de la longueur de l'ouverture latérale de la gaine sur la longueur de la cavité de la gaine est compris entre 0,17 et 0,67,
- un rapport de la largeur de l'ouverture latérale de la gaine sur la largeur de la cavité de la gaine est compris entre 0,24 et 0,67.

Selon une variante, le dispositif d'implantation est spécialement adapté pour un ballon intra-gastrique sphérique présentant un diamètre compris entre 8 cm et 10 cm à l'état gonflé, et :
- la longueur de la cavité de la gaine est comprise entre 12 cm et 18 cm, et la largeur de la cavité de la gaine est comprise entre 1,5 cm et 2,5 cm,
- la longueur de l'ouverture latérale de la gaine est comprise entre 3 cm et 8 cm et la largeur de l'ouverture latérale de la gaine est comprise entre 0,6 cm et 1,0 cm.

Selon une variante, la gaine est un cylindre selon l'axe longitudinal, de section circulaire.

Cette forme de la gaine permet de limiter le risque de formation de lésions dans les voies naturelles du corps humain dans lesquelles la gaine est insérée.

Selon une variante, l'extrémité d'introduction de la gaine est convexe extérieurement à la cavité, de préférence hémisphérique, de sorte à permettre l'introduction du dispositif d'implantation dans une voie naturelle du corps humain.

Cette forme convexe de la gaine, voire hémisphérique permet de faciliter l'insertion de la gaine dans les voies naturelles du corps humain en limitant le risque de lésions.

Selon un deuxième aspect de l'invention, il est proposé un système de traitement de l'obésité d'un individu comprenant :
- un dispositif d'implantation selon le premier aspect de l'invention, et
- un ballon intra-gastrique gonflable présentant un état gonflé et un état dégonflé.

Selon une variante, le ballon intra-gastrique est en silicone. Le silicone permet au ballon intra-gastrique d'être déformable sans se déchirer.

Selon une variante, le ballon intra-gastrique comprend une valve configurée pour coopérer avec l'embout du canal et présentant un état passant permettant de faire passer le fluide et un état bloquant permettant de bloquer le fluide.

A l'état passant, la valve autorise le passage du fluide depuis le canal jusqu'au ballon intra-gastrique, permettant ainsi de faire passer le ballon intra-gastrique de l'état dégonflé à l'état gonflé. A l'état bloquant, la valve empêche le fluide situé dans le ballon intra-gastrique de sortir du ballon intra-gastrique. La valve peut par exemple être unidirectionnelle et présenter un sens passant dans lequel le fluide peut circuler depuis le canal jusqu'au ballon intra-gastrique et un sens bloquant dans lequel elle empêche le fluide de circuler, empêchant le fluide situé dans le ballon intra-gastrique de sortir du ballon intra-gastrique.

L'invention peut être mise en oeuvre dans un procédé pour le traitement de l'obésité d'un individu.

En particulier, le système de traitement selon le deuxième aspect de l'invention peut être mis en oeuvre dans un procédé pour le traitement de l'obésité d'un individu, dans lequel
- le ballon intra-gastrique à l'état dégonflé est attaché à l'embout du canal du dispositif d'implantation,
- la gaine du dispositif d'implantation est insérée dans l'estomac de l'individu,
- le canal est alimenté par un fluide et le canal conduit le fluide pour gonfler le ballon intra-gastrique,
- le ballon intra-gastrique sort de la gaine dans l'estomac par l'ouverture latérale de la paroi latérale de la gaine, et est détaché de l'embout à l'état gonflé,
- la gaine du dispositif d'implantation est retirée de l'estomac de l'individu.

Selon une variante, pour attacher le ballon intra-gastrique à l'état dégonflé à l'embout du canal, le cathéter est coulissé dans le fourreau pour faire sortir l'embout de la cavité de la gaine.

Selon une variante, le ballon intra-gastrique prend appui sur la surface d'appui de la paroi latérale de la gaine lorsque le ballon intra-gastrique passe de l'état dégonflé à l'état gonflé.

Selon une variante, le ballon intra-gastrique sort automatiquement de la gaine par l'ouverture latérale lorsque le ballon intra-gastrique passe de l'état dégonflé à l'état gonflé.

Selon une variante, la gaine du dispositif d'implantation est insérée par l'extrémité d'introduction dans une voie naturelle du corps de l'individu.

### Brève description des dessins

D'autres caractéristiques, détails et avantages apparaîtront à la lecture de la description détaillée ci-après, et à l'analyse des dessins annexés, sur lesquels :
[Fig. 1] La figure 1 montre un système de traitement de l'obésité d'un individu selon l'invention comprenant un dispositif d'implantation comprenant une gaine et un ballon intra-gastrique gonflable, le ballon intra-gastrique étant positionné dans la gaine dans un état dégonflé ;
[Fig. 2] La figure 2 montre le dispositif d'implantation du système de traitement de l'obésité de la figure 1 ;
[Fig. 3] La figure 3 montre le ballon intra-gastrique du système de traitement de l'obésité de la figure 1, à l'état dégonflé ;
[Fig. 4] La figure 4 montre le ballon intra-gastrique du système de traitement de l'obésité de la figure 1 à l'état gonflé ;
[Fig. 5] La figure 5 montre le système de traitement de l'obésité de la figure 1 comprenant un canal comportant un fourreau et un cathéter coulissant dans le fourreau, et dans lequel le cathéter a été coulissé dans le fourreau pour que l'embout sorte de la cavité de la gaine ;
[Fig. 6] La figure 6 montre une vue agrandie de la zone référencée VI sur le système de traitement de l'obésité de la figure 1 ;
[Fig. 7] La figure 7 montre le système de traitement de l'obésité de la figure 1 dans lequel le dispositif d'implantation comporte un embout, l'embout étant dans une position de gonflage, et dans lequel le ballon intra-gastrique est attaché à l'embout, est entre l'état dégonflé à l'état gonflé et sort partiellement par une ouverture latérale de la gaine ;
[Fig. 8] La figure 8 montre le système de traitement de l'obésité de la figure 1 dans lequel le ballon intra-gastrique est détaché de l'embout et est dans l'état gonflé.

### Description des modes de réalisation

Sur les figures, les mêmes références désignent des éléments identiques ou analogues.

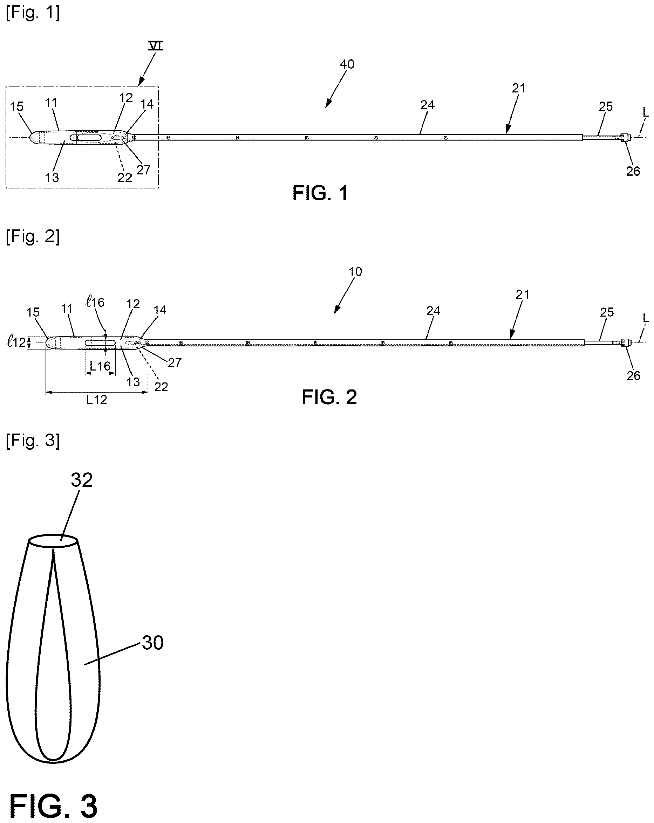

La figure 1 représente un système de traitement 40 de l'obésité d'un individu comprenant un dispositif d'implantation 10 et un ballon intra-gastrique 30 gonflable.

Le dispositif d'implantation 10 permet d'implanter le ballon intra-gastrique 30 gonflable dans l'estomac de l'individu. En particulier, l'implantation peut se faire par voie endoscopique, par une voie naturelle du corps de l'individu, comme par exemple par sa bouche et son oesophage.

Le ballon intra-gastrique 30 est en silicone. Alternativement, il pourrait être en tout autre matériau déformable élastiquement et biocompatible.

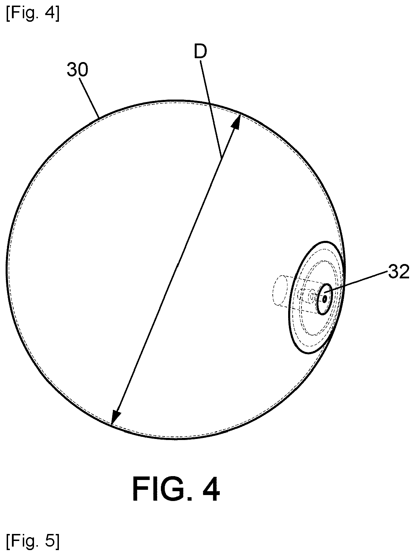

Le ballon intra-gastrique 30 présente un état dégonflé représenté à la figure 3 et un état gonflé représenté à la figure 4. Le ballon intra-gastrique 30 est implanté dans l'estomac de l'individu à l'état gonflé. Le ballon intra-gastrique 30 passe de l'état dégonflé à l'état gonflé en étant rempli d'un fluide tel que du sérum physiologique, de l'air ou un mélange de sérum physiologique et d'air.

Le ballon intra-gastrique 30 comprend une valve 32 présentant un état passant permettant de faire passer le fluide et un état bloquant permettant de bloquer le fluide. A l'état passant, la valve 32 autorise le fluide à entrer dans le ballon intra-gastrique 30, ce qui permet de gonfler le ballon intra-gastrique 30. A l'état bloquant, la valve 32 empêche le fluide situé dans le ballon intra-gastrique 30 de sortir du ballon intra-gastrique 30. La valve 32 permet donc de maintenir le ballon intra-gastrique 30 à l'état gonflé dans l'estomac de l'individu.

A l'état dégonflé, le ballon intra-gastrique 30 est replié sur lui-même. A l'état gonflé, le ballon intra-gastrique 30 est sphérique et présente un diamètre D. Alternativement, il pourrait prendre une forme ovoïdale ou ellipsoïdale.

Le dispositif d'implantation 10 est représenté à la figure 2. Il comprend une gaine 11 et un canal 21. La gaine 11 permet de recevoir le ballon intra-gastrique 30, et le canal 21 permet d'attacher le ballon intra-gastrique 30 et de le positionner dans la gaine 11. Le canal 21 peut être alimenté par un fluide et permet de conduire le fluide jusqu'au ballon intra-gastrique 30 pour gonfler le ballon intra-gastrique 30.

La gaine 11 est élastiquement déformable. La gaine 11 est cylindrique selon un axe longitudinal L. Elle comprend une paroi latérale 13 s'étendant autour de l'axe longitudinal entre une extrémité de raccordement 14 et une extrémité d'introduction 15. La section de la paroi latérale 13 par un plan perpendiculaire à l'axe longitudinal L est une section circulaire. Alternativement, la section pourrait être une section elliptique ou une section ovale. La gaine 11 est ainsi dépourvue d'arrêtes. Lorsque la gaine 11 est introduite par une voie naturelle du corps humain, elle est ainsi moins susceptible de créer des lésions dans cette voie naturelle du corps humain.

L'extrémité d'introduction 15 de la gaine 11 est hémisphérique. Elle pourrait alternativement prendre une autre forme convexe, par exemple une portion d'un hémisphère. Cette forme facilite l'insertion du dispositif d'implantation 10 par voie endoscopique en limitant le risque de formation de lésions dans la voie naturelle du corps humain dans laquelle le dispositif d'implantation est introduit.

La paroi latérale 13 présente une ouverture latérale 16. L'ouverture latérale 16 a une forme oblongue selon l'axe longitudinal L. Elle présente deux côtés parallèles entre eux et à l'axe longitudinal L et de même longueur. Chaque extrémité d'un des deux côtés est jointe à une des extrémités de l'autre côté par un arc de cercle.

La paroi latérale 13 présente une surface d'appui 17, opposée à l'ouverture latérale 16 et configurée pour que le ballon intra-gastrique 30 prenne appui dessus lorsque le ballon intra-gastrique 30 passe de l'état dégonflé à l'état gonflé. La surface d'appui 17 guide le ballon intra-gastrique 30 vers l'ouverture latérale 16.

La gaine 11 délimite une cavité 12 permettant de recevoir le ballon intra-gastrique 30 dans l'état dégonflé. Lorsque le ballon intra-gastrique 30 passe de l'état dégonflé à l'état gonflé, c'est-à-dire lorsqu'il est en cours de gonflage, l'ouverture latérale 16 de la paroi latérale 13 de la gaine 11 permet de laisser sortir le ballon intra-gastrique de la cavité.

La cavité 12 de la gaine 11 présente une longueur L12 prise selon l'axe longitudinal L et une largeur 112 prise perpendiculairement à l'axe longitudinal. La longueur L12 de la cavité 12 de la gaine 11 est prise depuis l'extrémité de raccordement 14 jusqu'à l'extrémité d'introduction 15.

L'ouverture latérale 16 présente une longueur L16 prise selon l'axe longitudinal L et une largeur l16 prise perpendiculairement à l'axe longitudinal L. La longueur L16 de l'ouverture latérale est prise depuis un sommet S1 d'un des arcs de cercle joignant les extrémités des deux côtés parallèles de l'ouverture latérale 16 à un sommet S2 de l'autre arc de cercle joignant les extrémités des deux côtés parallèles de l'ouverture latérale 16.

Le sommet S2 forme un bord d'appui sur lequel le ballon intra-gastrique 30 peut s'appuyer afin de permettre le détachement du ballon intra-gastrique 30 du canal 21, évitant ainsi que le ballon intra-gastrique 30 ne s'appuie sur une paroi de l'estomac de l'individu pour se détacher du canal 21.

Le canal 21 est attaché à l'extrémité de raccordement 14 de la gaine 11. Dans cet exemple, le canal 21 comprend un fourreau 24 attaché à l'extrémité de raccordement 14 de la gaine 11 et un cathéter 25 s'étendant à l'intérieur du fourreau 24. Le cathéter 25 est monté coulissant par rapport au fourreau 24. Il comporte une extrémité proximale 26 et une extrémité distale 27 opposée à l'extrémité proximale 26. L'extrémité distale 27 est en saillie du fourreau 24. Dans cet exemple, l'extrémité proximale 26 du cathéter 25 est alimentée en fluide et le cathéter 25 conduit le fluide jusqu'à l'extrémité distale 27. Dans un autre exemple non représenté, le canal 21 pourrait être constitué uniquement par un cathéter.

L'extrémité distale 27 comporte un embout 22. L'embout 22 permet d'attacher le ballon intra-gastrique 30 de manière amovible. En particulier, à l'état dégonflé, le ballon intra-gastrique 30 peut être attaché à l'embout 22 et à l'état gonflé, le ballon intra-gastrique 30 peut être détaché de l'embout 22.

L'embout 22 est représenté dans une position de gonflage. L'embout 22 se trouve dans cette position de gonflage notamment lorsque le ballon intra-gastrique 30 passe de l'état dégonflé à l'état gonflé. Dans cette position de gonflage, l'embout 22 s'étend dans la cavité 12 de la gaine 11. En particulier, l'embout 22 s'étend selon l'axe longitudinal L et est positionné entre l'extrémité de raccordement 14 de la gaine 11 et l'ouverture latérale 16 de la gaine 11 selon l'axe longitudinal L. Ce positionnement permet au ballon intra-gastrique 30 de sortir automatiquement de la gaine par l'ouverture latérale 16 lorsque le ballon intra-gastrique 30 passe de l'état dégonflé à l'état gonflé.

Afin de conduire le fluide jusqu'au ballon intra-gastrique 30, l'embout 22 coopère avec la valve 32 du ballon intra-gastrique 30. L'embout 22 est emmanché de façon amovible dans la valve 32 du ballon intra-gastrique 30.

L'embout 22, s'étend selon un axe d'embout, et comporte un épaulement 23 s'étendant transversalement par rapport à l'axe d'embout. Cet épaulement 23 permet d'aider à emmancher le ballon intra-gastrique 30 sur l'embout 22 de façon amovible. En particulier, le ballon intra-gastrique 30 peut être emmanché à l'embout 22 à l'état dégonflé, il peut être maintenu emmanché à l'embout 22 du canal 21 en cours de gonflage et il peut être détaché de l'embout 22 du canal 21 à l'état gonflé.

A titre illustratif, dans un exemple particulier non limitatif, le système de traitement est mis en oeuvre avec les dimensions suivantes.

Un rapport du diamètre D du ballon intra-gastrique 30 sur la longueur L12 de la cavité 12 de la gaine 11 peut être compris entre 0,44 et 0,83. Un rapport du diamètre D du ballon intra-gastrique 30 sur la largeur l12 de la cavité 12 de la gaine 11 peut être compris entre 3,2 et 6,7. Un rapport de la longueur L16 de l'ouverture latérale 16 de la gaine 11 sur la longueur L12 de la cavité 12 de la gaine 11 peut être compris entre 0,17 et 0,67. Un rapport de la largeur l16 de l'ouverture latérale 16 de la gaine 11 sur la largeur l12 de la cavité de la gaine pourrait être compris entre 0,24 et 0,67.

Le diamètre D du ballon intra-gastrique 30 peut être compris entre 8 cm et 10 cm. Pour un tel diamètre D du ballon intra-gastrique, la longueur L12 de la cavité 12 de la gaine 11 peut être comprise entre 12 cm et 18 cm, et la largeur l12 de la cavité 12 de la gaine 11 peut être comprise entre 1,5 cm et 2,5 cm. Et, la longueur L16 de l'ouverture latérale 16 peut être comprise entre 3 cm et 8 cm, et la largeur l16 de l'ouverture latérale 16 peut être comprise entre 0,6 cm et 1,0 cm.

Dans un exemple de réalisation, le rapport du diamètre D du ballon intra-gastrique 30 sur la longueur L12 de la cavité 12 de la gaine 11 est égal à 0,6. Le rapport du diamètre D du ballon intra-gastrique 30 sur la largeur l12 de la cavité 12 de la gaine 11 est égal à 4,5. Le rapport de la longueur L16 de l'ouverture latérale 16 de la gaine 11 sur la longueur L12 de la cavité 12 de la gaine 11 est égal à 0,3. Le rapport de la largeur l16 de l'ouverture latérale de la gaine 11 sur la largeur l12 de la cavité de la gaine est égal à 0,4.

Dans un exemple de réalisation, le diamètre D du ballon intra-gastrique 30 est égal à 9 cm. La longueur L12 de la cavité 12 de la gaine 11 est égale à 15 cm et la largeur l12 de la cavité 12 de la gaine 11 est égale à 2 cm. La longueur L16 de l'ouverture latérale 16 est égale à 4,5 cm et la largeur l16 de l'ouverture latérale 16 est égale à 0,8 cm.

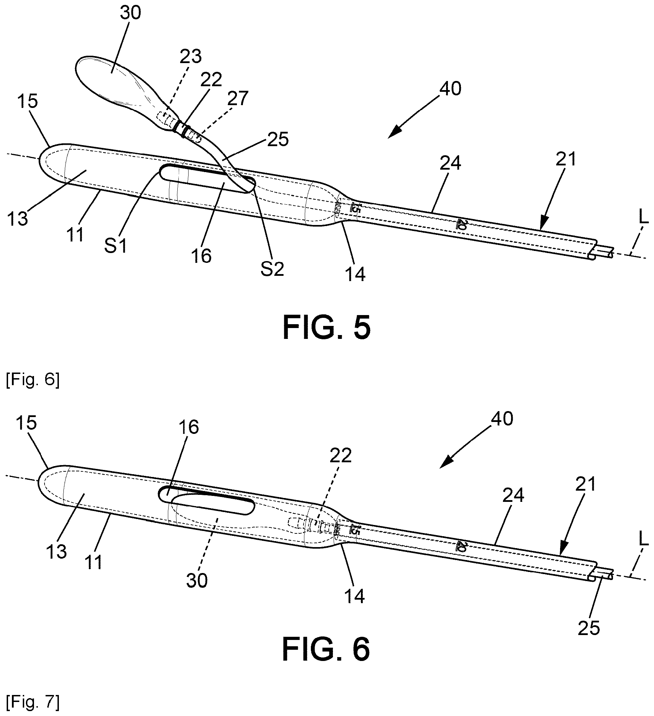

La mise en place du ballon intra-gastrique 30 sur l'embout 22 du canal 21 est décrite en relation avec la figure 5. Le cathéter 25 a été coulissé dans le fourreau 24 de sorte à ce que l'embout 22 passe dans l'ouverture latérale 16 de la gaine et sorte de la cavité 12 de la gaine 11. L'embout 22 est ainsi positionné hors de la cavité 12 de la gaine 11.

Le ballon intra-gastrique 30 est à l'état dégonflé. Il est attaché à l'embout 22 du canal 21 via la valve 32.

Le cathéter 25 peut ensuite être à nouveau coulissé dans le fourreau 24, de sorte à ce que l'embout 22 soit positionné dans la position de gonflage, tel que représenté à la figure 6. Le ballon intra-gastrique 30 est entièrement logé dans la gaine 11.

Le dispositif d'implantation 10 est alors inséré par l'extrémité d'introduction 15 de la gaine 11 dans la bouche et l'œsophage de l'individu jusqu'à ce que la gaine 11 atteigne l'estomac de l'individu.

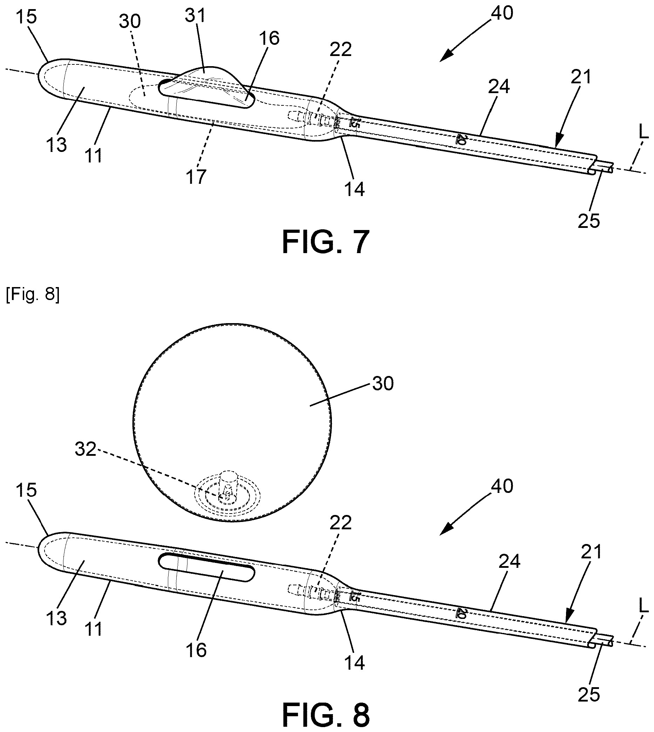

Comme représenté à la figure 7, le ballon intra-gastrique 30 est ensuite gonflé. Le canal 21 est alimenté à l'extrémité proximale 26 du cathéter 25 par un fluide, et le cathéter 25 conduit le fluide jusqu'au ballon intra-gastrique 30 par l'intermédiaire de l'embout 22 qui coopère avec la valve 32.

Le fluide est introduit dans la valve 32 à l'état passant. Le fluide dans le canal 21 vient créer une pression au niveau de l'embout 22, qui vient créer à son tour une pression sur la valve 32. La valve 32 est ainsi ouverte. Le ballon intra-gastrique 30 peut ainsi recevoir le fluide et être gonflé, pour passer de l'état dégonflé à l'état gonflé.

Lorsque le ballon intra-gastrique 30 est en cours de gonflage, c'est-à-dire lorsqu'il passe de l'état dégonflé à l'état gonflé, le ballon intra-gastrique 30 prend appui sur la surface d'appui 17 de la paroi latérale 13 de la gaine. La surface d'appui 17 guide le ballon intra-gastrique 30 vers l'ouverture latérale 16. Une partie 31 du ballon intra-gastrique 30 s'engage alors dans l'ouverture latérale 16 et sort automatiquement de la gaine 11 par l'ouverture latérale 16.

Une fois le ballon intra-gastrique 30 à l'état gonflé, celui-ci est détaché de l'embout 22. Le ballon intra-gastrique 30 peut par exemple être détaché de l'embout 22 suite au coulissement du cathéter 25 dans le fourreau 24 permettant de venir mettre en contact la ballon intra-gastrique 30 avec le bord d'appui formé par le sommet S2 de l'ouverture latérale 16. Le ballon intra-gastrique 30 est alors retenu hors de la gaine 11 en butant contre le bord d'appui et l'embout 22 est alors entrainé dans le fourreau 24, ce qui conduit au détachement du ballon intra-gastrique 30 de la gaine 11. Le ballon intra-gastrique 30 se retrouve ainsi hors de la gaine 11 et dissocié du dispositif d'implantation 10, tel que cela est représenté à la figure 8. Le ballon intra-gastrique 30 vient alors s'implanter dans l'estomac de l'individu, dans le fundus.

La valve 32 du ballon intra-gastrique 30, à l'état bloquant, empêche le fluide contenu dans le ballon intra-gastrique de sortir du ballon intra-gastrique 30, et le ballon reste à l'état gonflé dans l'estomac de l'individu.

Le dispositif d'implantation 10 est ensuite retiré de l'œsophage et de la bouche de l'individu.

## Revendications

1. Dispositif d'implantation (10) d'un ballon intra-gastrique (30) gonflable pour implanter le ballon intra-gastrique (30) dans un estomac, le ballon intra-gastrique (30) présentant un état dégonflé et un état gonflé, le dispositif d'implantation (10) comprenant :
- une gaine (11) déformable élastiquement et délimitant une cavité (12) configurée pour recevoir le ballon intra-gastrique (30) dans l'état dégonflé, la gaine (11) s'étendant selon un axe longitudinal (L) et comprenant une paroi latérale (13) s'étendant autour de l'axe longitudinal (L) entre une extrémité de raccordement (14) et une extrémité d'introduction (15),
- un canal (21) configuré pour être alimenté par un fluide et pour conduire le fluide pour gonfler le ballon intra-gastrique (30), le canal (21) étant attaché à l'extrémité de raccordement (14) de la gaine (11), et comportant un embout (22) configuré pour attacher le ballon intra-gastrique (30) de façon amovible, l'embout (22) présentant une position de gonflage dans laquelle ledit embout (22) s'étend dans la cavité (12) de la gaine (11),
et **caractérisé en ce que** la paroi latérale (13) de la gaine (11) présente une ouverture latérale (16) configurée pour laisser sortir le ballon intra-gastrique (30) de la cavité (12) lorsque ledit ballon intra-gastrique (30) passe de l'état dégonflé à l'état gonflé.

2. Dispositif d'implantation (10) selon la revendication 1, dans lequel le canal (21) comprend un fourreau (24) attaché à l'extrémité de raccordement (14) de la gaine (11) et un cathéter (25) s'étendant à l'intérieur du fourreau (24), le cathéter (25) étant monté coulissant par rapport au fourreau (24) et comportant une extrémité proximale (26) configurée pour être alimentée en fluide et une extrémité distale (27), l'extrémité distale (27) comportant l'embout (22) et étant en saillie du fourreau (24) dans la position de gonflage.

3. Dispositif d'implantation (10) selon la revendication 1 ou 2, dans lequel la paroi latérale (13) de la gaine (11) présente une surface d'appui (17), opposée à l'ouverture latérale (16) et configurée pour que le ballon intra-gastrique (30) prenne appui sur ladite surface d'appui (17) lorsque ledit ballon intra-gastrique (30) passe de l'état dégonflé à l'état gonflé.

4. Dispositif d'implantation (10) selon l'une des revendications 1 à 3, dans lequel l'ouverture latérale (16) de la gaine (11) présente un bord d'appui configuré pour que le ballon intra-gastrique (30) prenne appui sur ledit bord d'appui pour détacher le ballon intra-gastrique (30) de l'embout (22).

5. Dispositif d'implantation (10) selon l'une des revendications 1 à 4, dans lequel, dans la position de gonflage, l'embout (22) s'étend selon l'axe longitudinal (L) et est positionné entre l'extrémité de raccordement (14) de la gaine (11) et l'ouverture latérale (16) de la gaine (11) selon l'axe longitudinal L.

6. Dispositif d'implantation (10) selon l'une des revendications 1 à 5 spécialement adapté pour un ballon intra-gastrique (30) comportant une valve (32) configurée pour coopérer avec l'embout (22) du canal (21) et présentant un état passant permettant de faire passer le fluide et un état bloquant permettant de bloquer le fluide, et dans lequel l'embout (22) est configuré pour être emmanché de façon amovible dans la valve (32) du ballon intra-gastrique (30).

7. Dispositif d'implantation (10) selon la revendication 6, dans lequel l'embout (22) présente un axe d'embout et comprend au moins un épaulement (23) s'étendant transversalement par rapport à l'axe d'embout.

8. Dispositif d'implantation (10) selon l'une des revendications 1 à 7, dans lequel l'ouverture latérale (16) a une forme oblongue selon l'axe longitudinal (L).

9. Dispositif d'implantation (10) selon l'une quelconque des revendications 1 à 8 spécialement adapté pour un ballon intra-gastrique (30) sphérique présentant un diamètre D à l'état gonflé, et dans lequel la cavité (12) de la gaine (11) et l'ouverture latérale (16) présentent chacune une longueur (L12, L16) prise selon l'axe longitudinal (L) et une largeur (l12, l16) prise perpendiculairement à l'axe longitudinal (L) telles que :
- un rapport du diamètre D du ballon intra-gastrique (30) sur la longueur (L12) de la cavité (12) de la gaine (11) est compris entre 0,44 et 0,83,
- un rapport du diamètre D du ballon intra-gastrique (30) sur la largeur (l12) de la cavité (12) de la gaine (11) est compris entre 3,2 et 6,7,
- un rapport de la longueur (L16) de l'ouverture latérale (16) de la gaine (11) sur la longueur (L12) de la cavité (12) de la gaine (11) est compris entre 0,17 et 0,67,
- un rapport de la largeur de l'ouverture latérale de la gaine sur la largeur de la cavité de la gaine est compris entre 0,24 et 0,67.

10. Dispositif d'implantation (10) selon la revendication 9 spécialement adapté pour un ballon intra-gastrique (30) sphérique présentant un diamètre D compris entre 8 cm et 10 cm à l'état gonflé, dans lequel :
- la longueur (L12) de la cavité (12) de la gaine (11) est comprise entre 12 cm et 18 cm, et la largeur (112) de la cavité (12) de la gaine (11) est comprise entre 1,5 cm et 2,5 cm,
- la longueur (L16) de l'ouverture latérale (16) de la gaine (11) est comprise entre 3 cm et 8 cm et la largeur (l16) de l'ouverture latérale (16) de la gaine (11) est comprise entre 0,6 cm et 1 cm.

11. Dispositif d'implantation (10) selon l'une des revendications 1 à 10, dans lequel la gaine (11) est un cylindre selon l'axe longitudinal (L), de section circulaire.

12. Dispositif d'implantation (10) selon l'une des revendications 1 à 11, dans lequel l'extrémité d'introduction (15) de la gaine (11) est convexe extérieurement à la cavité (12), de préférence hémisphérique, de sorte à permettre l'introduction du dispositif d'implantation (10) dans une voie naturelle du corps humain.

13. Système de traitement (40) de l'obésité d'un individu comprenant :
- un dispositif d'implantation (10) selon l'une des revendications 1 à 12, et
- un ballon intra-gastrique (30) gonflable présentant un état gonflé et un état dégonflé.

14. Système de traitement (40) de l'obésité d'un individu selon la revendication 13, dans lequel le ballon intra-gastrique (30) comprend une valve (32) configurée pour coopérer avec l'embout (22) du canal (21) et présentant un état passant permettant de faire passer le fluide et un état bloquant permettant de bloquer le fluide.

## Patentansprüche

1. Implantationsvorrichtung (10) für einen aufblasbaren intragastrischen Ballon (30) zum Implantieren des intragastrischen Ballons (30) in einen Magen, wobei der intragastrische Ballon (30) einen entleerten Zustand und einen aufgeblasenen Zustand aufweist, wobei die Implantationsvorrichtung (10) umfasst:
- eine Hülle (11), die elastisch verformbar ist und einen Hohlraum (12) begrenzt, der dazu ausgebildet ist, den intragastrischen Ballon (30) im entleerten Zustand aufzunehmen, wobei sich die Hülle (11) entlang einer Längsachse (L) erstreckt und eine Seitenwand (13) umfasst, die sich um die Längsachse (L) zwischen einem Verbindungsende (14) und einem Einführsende (15) erstreckt,
- einen Kanal (21), der dazu ausgebildet ist, mit einem Fluid versorgt zu werden und das Fluid zum Aufblasen des intragastrischen Ballons (30) zu leiten, wobei der Kanal (21) an dem Verbindungsende (14) der Hülle (11) angebracht ist und ein Mundstück (22) aufweist, das dazu ausgebildet ist, den intragastrischen Ballon (30) lösbar zu befestigen,
wobei das Mundstück (22) eine Aufblasposition aufweist, in der sich das Mundstück (22) in den Hohlraum (12) der Hülle (11) hinein erstreckt,
und **dadurch gekennzeichnet, dass** die Seitenwand (13) der Hülle (11) eine seitliche Öffnung (16) aufweist, die dazu ausgebildet ist, den intragastrischen Ballon (30) aus dem Hohlraum (12) herauszulassen, wenn der intragastrische Ballon (30) vom entleerten Zustand in den aufgeblasenen Zustand übergeht.

2. Implantationsvorrichtung (10) nach Anspruch 1, wobei der Kanal (21) eine Hülse (24) umfasst, die am Verbindungsende (14) der Hülle (11) befestigt ist, und einen Katheter (25) umfasst, der sich in die Hülse (24) hinein erstreckt, wobei der Katheter (25) in Bezug auf die Hülse (24) verschiebbar angebracht ist und ein proximales Ende (26) aufweist, das dazu ausgebildet ist, mit Flüssigkeit versorgt zu werden, und ein distales Ende (27) aufweist, wobei das distale Ende (27) das Mundstück (22) umfasst und in der Aufblasposition aus der Hülse (24) herausragt.

3. Implantationsvorrichtung (10) nach Anspruch 1 oder 2, wobei die Seitenwand (13) der Hülle (11) eine Auflagefläche (17) aufweist, die der seitlichen Öffnung (16) gegenüberliegt und derart ausgebildet ist, dass der intragastrische Ballon (30) auf der Auflagefläche (17) aufliegt, wenn der intragastrische Ballon (30) vom entleerten Zustand in den aufgeblasenen Zustand übergeht.

4. Implantationsvorrichtung (10) nach einem der Ansprüche 1 bis 3, wobei die seitliche Öffnung (16) der Hülle (11) eine Auflagekante aufweist, die derart ausgebildet ist, dass der intragastrische Ballon (30) auf der Auflagekante aufliegt, um den intragastrischen Ballon (30) von dem Mundstück (22) zu lösen.

5. Implantationsvorrichtung (10) nach einem der Ansprüche 1 bis 4, wobei sich das Mundstück (22) in der Aufblasposition entlang der Längsachse (L) erstreckt und zwischen dem Verbindungssende (14) der Hülle (11) und der seitlichen Öffnung (16) der Hülle (11) entlang der Längsachse L positioniert ist.

6. Implantationsvorrichtung (10) nach einem der Ansprüche 1 bis 5, die speziell für einen intragastrischen Ballon (30) angepasst ist, der ein Ventil (32) aufweist, das dazu ausgebildet ist, mit dem Mundstück (22) des Kanals (21) zusammenzuwirken und einen Durchlasszustand aufweist, der das Durchlassen von Fluid ermöglicht, und einen Blockierzustand aufweist, der das Blockieren von Fluid ermöglicht, und wobei das Mundstück (22) dazu ausgebildet ist, abnehmbar in das Ventil (32) des intragastrischen Ballons (30) eingepasst zu werden.

7. Implantationsvorrichtung (10) nach Anspruch 6, wobei das Mundstück (22) eine Mundstückachse aufweist und wenigstens eine Schulter (23) umfasst, die sich quer zur Mundstückachse erstreckt.

8. Implantationsvorrichtung (10) nach einem der Ansprüche 1 bis 7, wobei die seitliche Öffnung (16) entlang der Längsachse (L) eine längliche Form aufweist.

9. Implantationsvorrichtung (10) nach einem der Ansprüche 1 bis 8, die speziell für einen kugelförmigen intragastrischen Ballon (30) angepasst ist, der im aufgeblasenen Zustand einen Durchmesser D aufweist, und wobei der Hohlraum (12) der Hülle (11) und die seitliche Öffnung (16) jeweils eine Länge (L12, L16) entlang der Längsachse (L) und eine Breite (l12, l16) senkrecht zur Längsachse (L), aufweisen, so dass:
- ein Verhältnis des Durchmessers D des intragastrischen Ballons (30) zur Länge (L12) des Hohlraums (12) der Hülle (11) zwischen 0,44 und 0,83 liegt,
- ein Verhältnis des Durchmessers D des intragastrischen Ballons (30) zur Breite (l12) des Hohlraums (12) der Hülle (11) zwischen 3,2 und 6,7 liegt,
- ein Verhältnis der Länge (L16) der seitlichen Öffnung (16) der Hülle (11) zur Länge (L12) des Hohlraums (12) der Hülle (11) zwischen 0,17 und 0,67 liegt,
- ein Verhältnis der Breite der Seitenöffnung der Hülle zur Breite des Hohlraums der Hülle zwischen 0,24 und 0,67 liegt.

10. Implantationsvorrichtung (10) nach Anspruch 9, welche speziell für einen kugelförmigen intragastrischen Ballon (30) mit einem Durchmesser D zwischen 8 cm und 10 cm im aufgeblasenen Zustand angepasst ist, wobei:
- die Länge (L12) des Hohlraums (12) der Hülle (11) zwischen 12 cm und 18 cm liegt, und die Breite (l12) des Hohlraums (12) der Hülle (11) zwischen 1,5 cm und 2,5 cm liegt,
- die Länge (L16) der seitlichen Öffnung (16) der Hülle (11) zwischen 3 cm und 8 cm liegt und die Breite (l16) der seitlichen Öffnung (16) der Hülle (11) zwischen 0,6 cm und 1 cm liegt.

11. Implantationsvorrichtung (10) nach einem der Ansprüche 1 bis 10, wobei die Hülle (11) entlang der Längsachse (L) ein Zylinder mit kreisförmigem Querschnitt ist.

12. Implantationsvorrichtung (10) nach einem der Ansprüche 1 bis 11, wobei das Einführende (15) der Hülle (11) außerhalb des Hohlraums (12) konvex, bevorzugt halbkugelförmig, ist, so dass die Implantationsvorrichtung (10) in einen natürlichen Weg des menschlichen Körpers eingeführt werden kann.

13. System (40) zur Behandlung von Adipositas bei einem Individuum, umfassend:
- eine Implantationsvorrichtung (10) nach einem der Ansprüche 1 bis 12, und
- einen aufblasbaren intragastrischen Ballon (30), der einen aufgeblasenen Zustand und einen entleerten Zustand aufweist.

14. System zur Behandlung (40) von Adipositas bei einem Individuum nach Anspruch 13, wobei der intragastrische Ballon (30) ein Ventil (32) umfasst, das dazu ausgebildet ist, mit dem Mundstück (22) des Kanals (21) zusammenzuwirken und einen Durchlasszustand aufweist, der das Durchlassen von Fluid ermöglicht, und einen Blockierzustand aufweist, der das Blockieren von Fluid ermöglicht.

## Claims

1. Placement device (10) for an inflatable gastric balloon (30) for the placement of the gastric balloon (30) in a stomach, the gastric balloon (30) having a deflated state and an inflated state, the placement device (10) comprising:
- an elastically deformable casing (11) delimiting a cavity (12) configured to receive the gastric balloon (30) in the deflated state, the casing (11) extending along a longitudinal axis (L) and comprising a lateral wall (13) extending around the longitudinal axis (L) between a connection end (14) and an insertion end (15),
- a duct (21) configured to be supplied with a fluid and to convey the fluid to inflate the gastric balloon (30), the duct (21) being attached to the connection end (14) of the casing (11), and comprising an end piece (22) configured to removably attach the gastric balloon (30);
- the end piece (22) having an inflation position in which said end piece (22) extends into the cavity (12) of the casing (11);
**characterized in that** the lateral wall (13) of the casing (11) has a lateral opening (16) configured to allow the gastric balloon (30) to exit the cavity (12), when said gastric balloon (30) passes from the deflated state to the inflated state.

2. Placement device (10) according to claim 1, wherein the duct (21) comprises a sheath (24) attached to the connection end (14) of the casing (11) and a catheter (25) extending inside the sheath (24), the catheter (25) being mounted with the ability to slide with respect to the sheath (24) and comprising a proximal end (26) configured to be supplied with fluid and a distal end (27), the distal end (27) comprising the end piece (22) and projecting from the sheath (24) in the inflation position.

3. Placement device (10) according to claim 1 or 2, wherein the lateral wall (13) of the casing (11) has a bearing surface (17), opposite the lateral opening (16) and configured so that the gastric balloon (30) bears against said bearing surface (17) when said gastric balloon (30) passes from the deflated state to the inflated state.

4. Placement device (10) according to one of claims 1 to 3, wherein the lateral opening (16) of the casing (11) has a bearing edge configured, so that the gastric balloon (30) bears against said bearing edge for detaching the gastric balloon (30) from the end piece (22).

5. Placement device (10) according to one of claims 1 to 4, wherein, in the inflation position, the end piece (22) extends along the longitudinal axis (L) and is positioned between the connection end (14) of the casing (11) and the lateral opening (16) of the casing (11) along the longitudinal axis L.

6. Placement device (10) according to one of claims 1 to 5, specially designed for a gastric balloon (30) comprising a valve (32) configured to collaborate with the end piece (22) of the duct (21) and having an open state allowing fluid to pass and a closed state blocking the fluid, and wherein the end piece (22) is configured to be push-fitted removably into the valve (32) of the gastric balloon (30).

7. Placement device (10) according to claim 6, wherein the end piece (22) has an end piece axis and comprises at least one shoulder (23) extending transversely with respect to the end piece axis.

8. Placement device (10) according to one of claims 1 to 7, wherein the lateral opening (16) has a shape that is oblong along the longitudinal axis (L).

9. Placement device (10) according to one of claims 1 to 8, specially designed for a spherical gastric balloon (30) having a diameter D in the inflated state, and wherein the cavity (12) of the casing (11) and the lateral opening (16) each have a length (L12, L16) considered along the longitudinal axis (L) and a width (112, 116) considered perpendicular to the longitudinal axis (L) such that:
- a ratio of the diameter D of the gastric balloon (30) to the length (L12) of the cavity (12) of the casing (11) is comprised between 0.44 and 0.83,
- a ratio of the diameter D of the gastric balloon (30) to the width (112) of the cavity (12) of the casing (11) is comprised between 3.2 and 6.7,
- a ratio of the length (L16) of the lateral opening (16) of the casing (11) to the length (L12) of the cavity (12) of the casing (11) is comprised between 0.17 and 0.67,
- a ratio of the width of the lateral opening of the casing to the width of the cavity of the casing is comprised between 0.24 and 0.67.

10. Placement device (10) according to claim 9, specially designed for a spherical gastric balloon (30) having a diameter D comprised between 8 cm and 10 cm in the inflated state, wherein:
- the length (L12) of the cavity (12) of the casing (11) is comprised between 12 cm and 18 cm, and the width (112) of the cavity (12) of the casing (11) is comprised between 1.5 cm and 2.5 cm,
- the length (L16) of the lateral opening (16) of the casing (11) is comprised between 3 cm and 8 cm and the width (116) of the lateral opening (16) of the casing (11) is comprised between 0.6 cm and 1 cm.

11. Placement device (10) according to one of claims 1 to 10, wherein the casing (11) is a cylinder along the longitudinal axis (L), of circular cross section.

12. Placement device (10) according to one of claims 1 to 11, wherein the insertion end (15) of the casing (11) is convex towards the outside of the cavity (12), preferably hemispherical, so that the placement device (10) can be introduced into a natural tract of the human body.

13. System (40) for treating obesity in an individual, comprising:
- a placement device (10) according to one of claims 1 to 12, and
- an inflatable gastric balloon (30) having an inflated state and a deflated state.

14. System (40) for treating obesity in an individual according to claim 13, wherein the gastric balloon (30) comprises a valve (32) configured to collaborate with the end piece (22) of the duct (21) and having an open state allowing fluid to pass and a closed state blocking the fluid.
